# EUROPÄISCHE PATENTANMELDUNG

(11) **EP 3 461 906 A1**
(43) Veröffentlichungstag der Anmeldung: **03.04.2019**
(21) Anmeldenummer: 17193973.9
(22) Anmeldetag: 29.09.2017
(51) Int. Cl.: C12Q 1/22, A61L 2/28

(54) **VORRICHTUNG UND VERFAHREN ZUR MIKROBIOLOGISCHEN PRÜFUNG VON WASCHMASCHINEN**

(71) Anmelder: Meducomp GmbH, 15378 Herzfelde (DE)
(72) Erfinder: Cyganek, Jürgen, 15378 Herfelde (DE)
(74) Vertreter: Hertin und Partner Rechts- und Patentanwälte PartG mbB

(57) **Zusammenfassung**

Die Erfindung betrifft einen Bioindikator und ein Verfahren zur Verwendung des Bioindikators zur mikrobiologischen Prüfung von Waschmaschinen. Die erfindungsgemäße Vorrichtung erlaubt eine sichere, zuverlässige, kosteneffiziente und leicht handhabbare mikrobiologische Prüfung der Desinfektionsleistung von Waschmaschinen. Die Erfindung betrifft außerdem eine Verwendung des Bioindikators und einen Kit umfassend 10 Bioindikatoren. Insbesondere betrifft die Erfindung einen geschlossenen Bioindikator zur Überprüfung von desinfizierenden Waschverfahren.

## Beschreibung

Die Erfindung betrifft einen Bioindikator und ein Verfahren zur Verwendung des Bioindikators zur mikrobiologischen Prüfung von Waschmaschinen. Die erfindungsgemäße Vorrichtung erlaubt eine sichere, zuverlässige, kosteneffiziente und leicht handhabbare mikrobiologische Prüfung der Desinfektionsleistung von Waschmaschinen. Die Erfindung betrifft außerdem eine Verwendung des Bioindikators und einen Kit, der bevorzugt 10 Bioindikatoren umfasst. Insbesondere betrifft die Erfindung einen geschlossenen Bioindikator zur Überprüfung von desinfizierenden Vorgängen wie beispielsweise Spül- oder Waschverfahren, insbesondere in Wasch- oder Spülmaschinen bzw. allen Vorrichtungen, mithilfe derer ein Desinfektionsvorgang durchgeführt bzw. nachgewiesen werden kann. Hierbei kann es sich z.B. um Maschinen mit Desinfektionsprogrammen handeln.

### Hintergrund und Stand der Technik

Zur Vorbeugung vor Krankheiten ist es entscheidend, in sensiblen Umfeldern, wie beispielsweise Krankenhäusern, höchsten Anforderungen an die Hygiene gerecht zu werden. Desinfektionsgeräte wie Wasch- und Spülmaschinen müssen regelmäßig auf ihre Desinfektionsleistung überprüft werden. Auch Spülmaschinen müssen in sensiblen Bereichen ihre Desinfektionsleistung nachweisen.

Funktioniert eine Waschmaschine nicht einwandfrei, kann sie zu einem Ansiedlungspunkt und einer Vermehrungsstätte für Krankheitserreger wie beispielsweise einer Vielzahl von Bakterien werden. Diese können dann beispielsweise von einem eingebrachten, infizierten Gut, wie beispielsweise Krankenhausbettwäsche, auf alle zu waschenden und zu desinfizierenden Güter übergehen. Ein Waschvorgang mit einer defekten oder fehlerhaft bedienten Waschmaschine bietet den Mikroorganismen somit eine ideale Verbreitungsmöglichkeit. Um dies zu verhindern, muss sichergestellt werden, dass die Waschmaschine einwandfrei funktioniert, d.h., dass ihre Desinfektionsleistung den Anforderungen entspricht. Die Desinfektionsleistung wird durch viele Faktoren beeinflusst. Eine unzureichende Desinfektionsleistung kann beispielsweise durch Bedienungsfehler, Fehlprogrammierung oder Fehldosierung von Wirkstoffen oder aber durch ein defektes Desinfektionsgerät hervorgerufen werden.

Deswegen kontrollieren Gesundheitsämter regelmäßig, ob Prüfungen von Desinfektionsgeräten durchgeführt wurden. Auch eine Reihe von Richtlinien von für die Gesundheit zuständigen Instituten, wie zum Beispiel dem Robert-Koch-Institut für Infektionskrankheiten, fordern die regelmäßige Überprüfung solcher Geräte.

Eine Überprüfung der Desinfektionsleistung kann auf mehrere Arten erfolgen. Beispielsweise kann über ein Datenaufzeichnungsgerät direkt die Temperatur des Desinfektionsgeräts aufgezeichnet werden. Bei einigen Desinfektionsverfahren können so Rückschlüsse auf den Desinfektionserfolg gezogen werden. Dieses Verfahren ist jedoch hauptsächlich für rein thermische Desinfektionsverfahren geeignet. Außerdem erfordert diese Methode gegebenenfalls eine teure Nachrüstung der Datenaufzeichnungsgeräte.

Es ist auch möglich, die Desinfektionsleistung mittels sogenannter Abklatschproben zu überprüfen. Dabei wird das zu desinfizierenden Gut nach dem Desinfektionsvorgang mit einem sich in einem Plastikschälchen befindenden Nährboden in Berührung gebracht. Im Labor wird dann untersucht, ob ein Keimwachstum auf dem Nährboden stattgefunden hat. Diese Methode ist jedoch aufwendig, nur von geschultem Fachpersonal durchzuführen und fehleranfällig.

Eine weitere Art der Überprüfung geschieht mittels sogenannter Bioindikatoren. In zahlreichen Ländern wie bspw. Deutschland oder Österreich gibt es hierfür Vorschriften bzw. Richtlinien. Dabei wird ein Keim in einer bestimmten Keimdichte in die Maschine eingebracht. Nach dem Desinfektionslauf, beispielsweise dem Waschgang, wird überprüft, ob und in welcher Größenordnung eine Keimreduktion stattgefunden hat.

Insbesondere Desinfektionsgeräte, welche mit sogenannter Risikowäsche in Berührung kommen, in Krankenhäusern und Altenheim, müssen mindestens halbjährlich überprüft werden.

Bekannt sind Prüfungsmethoden mittels Bioindikatoren durch sogenannte offene Systeme, bei denen man die Keime des Bioindikators direkt in die Waschmaschine einbringt. Beispielsweise kann ein kleines, mit einem geeigneten Indikator versehenes Baumwollsäckchen verwendet werden. Diese Bioindikatoren haben den Vorteil, dass zur Durchführung der Prüfung kein Fachpersonal nötig ist. Solange die Waschmaschine einwandfrei funktioniert, findet eine Keimreduktion statt. Die bekannten Verfahren weisen jedoch mehrere Nachteile auf. Funktioniert ein Gerät nicht einwandfrei, wird das gesamte Waschgut kontaminiert. Somit stellt der Prüfungsvorgang selber ein Gesundheitsrisiko dar. Des Weiteren kann dabei trotz der nicht stattgefundenen Desinfektion eine Verdünnung der Keimdichte auf dem Bioindikator stattfinden, d.h. das Prüfungsergebnis könnte trotzdem positiv sein. Ebenso kann bei diesem Verfahren trotz erfolgreicher Desinfektion der Bioindikator nach der Prüfung durch unsachgemäße Handhabung wieder verkeimt werden und das Prüfungsergebnis fällt negativ aus. Dies hat zu guter Letzt wiederholte Prüfungen und einen mit erhöhten Kosten verbundenen Mehraufwand zur Folge. Die Alltagsnutzung der Waschmaschine zur Desinfektion und Reinigung von Textilien muss dabei länger unterbrochen werden.

Außerdem gibt es in einzelnen Ländern Vorschriften, die mindestens drei Leerläufe der Desinfektionsvorrichtungen wie Geschirrspülern oder Waschmaschinen, nachdem sie zur Desinfektion eingesetzt wurden und das Ergebnis der Kontaminierungsprüfung nicht den Vorschriften genügt. Hierdurch kommt es zu einem großen Verlust an Ressourcen wie bspw. Trinkwasser oder Reinigungsmitteln.

### Aufgabe der Erfindung

Aufgabe der Erfindung ist eine Vorrichtung und ein Verfahren bereitzustellen, welches die Nachteile des Standes der Technik nicht aufweist.

Der Erfindung liegt insbesondere die Aufgabe zugrunde, ein Verfahren sowie eine Vorrichtung zur Überprüfung der Desinfektionsleistung von Desinfektionsgeräten, insbesondere Waschmaschinen, bereitzustellen, wobei das Verfahren und die Vorrichtung einfach und ohne die Anwesenheit von Fachpersonal anwendbar ist, und dabei die Gefahr einer fehlerhaften Durchführung minimiert bzw. ausschließt. Vor allem soll die mögliche Kontamination des gesamten Waschguts und daraus möglicherweise resultierende weitere Kontaminationen bei einer Überprüfung sowie das Risiko eines verfälschten Prüfungsergebnisses minimiert werden.

### Kennzeichnung der Erfindung

Erfindungsgemäß wird die Aufgabe durch eine Vorrichtung gemäß den unabhängigen Patentansprüchen gelöst. Die abhängigen Patentansprüche stellen bevorzugte Ausführungsformen der Erfindung dar.

In einem Aspekt betrifft die Erfindung einen Bioindikator zur mikrobiologischen Prüfung von Waschmaschinen, wobei
- der Bioindikator einen Träger umfasst,
- der Träger Referenzkeime aufweist, wobei
- der Träger durch eine semipermeable Membran umschlossen ist.

Unter Bioindikator versteht man im Sinne der Erfindung bevorzugt eine Vorrichtung, welche über den Vergleich der Anzahl von Mikroorganismen in einem räumlich begrenzten Bereich vor und nach einem zu prüfenden Desinfektionsvorgang einen Aufschluss über den Erfolg des Desinfektionsvorgangs zulässt. Räumlich begrenzter Bereich bezeichnet im Sinne der Erfindung bevorzugt eine Fläche mit einer Ausdehnung zwischen 0,25 cm² und 100 cm² und umfasst bevorzugt einen relevanten Teil des Bioindikators. Mit Desinfektionsvorgang ist bevorzugt ein Vorgang gemeint, vormals pathogene Mikroorganismen in einen Zustand zu versetzen, in dem sie nicht mehr pathogen sind. Besonders bevorzugt dient der Desinfektionsvorgang dem Abtöten der Mikroorgansimen. Mit pathogen ist bevorzugt potentiell krankheitserregend gemeint. Besonders bevorzugt ist mit Desinfektionsvorgang im Sinne der Erfindung ein Waschvorgang einer Waschmaschine oder ein Spülvorgang einer Spülmaschine gemeint. Der Desinfektionsvorgang kann thermisch und/oder chemisch vorgenommen werden. Durch die Messung der Dichte von Mikroorganismen als Referenzkeimen, zum Beispiel durch die Messung der Dichte von Bakterien vor und nach einer Desinfektionshandlung, kann der Erfolg der Desinfektionshandlung gemessen werden. Mit Dichte bzw. Keimdichte ist bevorzugt die Anzahl pro Fläche gemeint, besonders bevorzugt ist die Anzahl pro cm² gemeint. Es kann aber auch bevorzugt sein, dass die Anzahl der Keime pro Bioindikator gemeint ist. Erfolg bezeichnet bevorzugt eine Keimreduktion um mehrere Größenordnungen, also bevorzugt um einen Faktor von mehreren Zehnerpotenzen. Typischerweise soll bei einer erfolgreichen Keimreduktion eine Reduktion der Keimdichte um bevorzugt mindestens drei Größenordnungen oder Log-Stufen, insbesondere um mindestens vier Größenordnungen oder Log-Stufen, ganz besonders bevorzugt um mindestens fünf Größenordnungen oder Log-Stufen, in manchen Fällen bevorzugt mindestens sechs Größenordnungen oder Log-Stufen erreicht werden. Zum Nachweis der Keimreduktion bzw. zur Messung der Keimdichte können gängige Verfahren, wie beispielsweise bevorzugt die Gram-Färbung, lichtmikroskopische Untersuchungen, aber auch das Anwachsen einer Kultur auf einem Nährboden, verwendet werden.

Unter mikrobiologischer Prüfung wird bevorzugt eine Prüfung bezüglich Mikroorganismen, also Organismen, die sehr klein sind und aus einer bis wenigen Zellen bestehen, bezeichnet. Unter Mikroorganismen werden im Sinne der Erfindung bevorzugt Bakterien, Pilze und Viren bezeichnet. Hierbei sind insbesondere pathogene Mikroorganismen von Interesse. Bakterien sind im Sinne der Erfindung alle Prokaryoten, bei denen die DNA frei im Cytoplasma vorliegt. Bakterien gehören im Sinne der Erfindung bevorzugt zu den folgenden Stämmen: Actinobacteria, Firmicutes, Tenericutes, Aquificae, Bacteroidetes, Fibrobacteres, Chlorobi, Chlamydiae, Deinococcus-Thermus, Fusobacteria, Gemmatimonadetes, Nitrospirae, Planctomyceten, Verrucomicrobia, Chlamydiae, Proteobacteria, Spirochaetes, Synergistetes, Acidobacteria, Chloroflexi, Chrysiogenetes, Cyanobakterium, Deferribacteres, Dictyoglomi, Thermodesulfobacteria und/oder Thermotogae.

Unter den pilzartigen Mikroorganismen sind bevorzugt folgende Pilze zu verstehen: Hefen, wie beispielsweise Malassezia furfur und Candida albicans, Dermatophyten, verschiedene Aspergillus-Arten, zum Beispiel Aspergillus fumigatus. Außerdem können insbesondere Pilze der Gattung Cryptococcus, Rhizopus, Coccidioides und/oder Histoplasma pilzartige Mikroorganismen sein.

Viren sind bevorzugt infektiöse Partikel, die sich als Virionen außerhalb von Zellen (extrazellulär) durch Übertragung verbreiten, aber als Viren vor allem innerhalb einer geeigneten Wirtszelle (intrazellulär) vermehren können. Die bei der mikrobiologischen Prüfung im Sinne der Erfindung relevanten Viren sind bevorzugt Viren der Gattungen Orthopoxvirus, Parapoxvirus, Molluscipoxvirus, Simplexvirus, Varicellovirus, Cytomegalovirus, Reseolovirus, Lymphocryptovirus, Rhadinovirus, Orthohepadnavirus, Rubiviren, Flavivirus, Alphacoronavirus, Torovirus, Deltaretrovirus, Lentivirus, Bornavirus, Orthobunyavirus, Phlebovirus, Nairovirus, Hantavirus, Influenzavirus A, Influenzavirus B, Influenzavirus C, Avulavirus, Morbillivirus, Henipavirus, Rubulaviren, Pneumovirus, Metapneumovirus, Vesiculovirus, Mastadenovirus, Polyomavirus, Dependovirus, Erythrovirus, Rotavirus, Coltivirus, Norovirus, Sapovirus, Hepevirus, Enterovirus, Hepatovirus und/oder Rhinovirus.

Unter Prüfung ist bevorzugt eine Kontrolle auf Vorhandensein eines Mikroorganismus und/oder von Teilen eines Mikroorganismus zu verstehen. Es ist bevorzugt, dass bei der Kontrolle auf Vorhandensein die Anzahl und/oder die Dichte der Mikroorganismen bestimmt werden kann. Bevorzugt kann die Anzahl und/oder die Dichte vor und nach dem zu überprüfenden Desinfektionsvorgang und somit die Keimreduktion bestimmt werden. Es kann auch bevorzugt sein, dass vor dem Desinfektionsvorgang eine bekannte Anzahl an Mikroorganismen vorhanden ist, und nach dem Desinfektionsvorgang die Anzahl der Mikroorganismen bestimmt werden kann. Die Überprüfung kann beispielsweise durch Gram-Färbung, lichtmikroskopische Untersuchungen, aber auch das Anwachsen einer Kultur auf einem Nährboden stattfinden.

Eine Waschmaschine dient der Reinigung von Waschgütern. Bei diesem Reinigungsprozess wirken bevorzugt mechanische Kräfte und Wasser kombiniert auf das zu waschende Gut ein. Dem Waschwasser werden bevorzugt Waschmittel zugegeben, die bevorzugt ebenso wie die Temperatur des Waschwassers an das Material des zu waschenden Guts und dessen Verschmutzungsgrad und Verschmutzungsart angepasst werden. Waschmaschinen sind im klinischen Bereich verbreitet, um Bekleidung und andere textile Fertigerzeugnisse zu reinigen und bevorzugt zu desinfizieren.

Unter Träger sind bevorzugt flächige Elemente zu verstehen, welche zur Aufnahme von Referenzkeimen geeignet sind. Ein Träger kann verschiedenste Beschaffenheit aufweisen, beispielsweise kann er starr aber auch flexibel sein. Ebenso sind verschiedenste Geometrien, beispielsweise rund, viereckig, insbesondere quadratisch, aber ebenso andere geometrische Formen denkbar. Unter flächig ist bevorzugt zu verstehen, dass das Element in zwei Dimensionen eine große Ausdehnung und in der dritten eine geringe Ausdehnung hat. Beispielsweise kann das Element in zwei Dimensionen eine Ausdehnung von jeweils mindestens 1 cm aufweisen, und dabei eine Dicke von nur wenigen Millimetern oder sogar kleiner als 1 mm aufweisen.

Verschiedenste Materialien sind als Trägermaterial denkbar. Es sind beispielsweise textile Materialien aus Naturfasern wie Baumwolle bevorzugt, aber auch Polymere sind als bevorzugtes Trägermaterial denkbar.

Bevorzugt ist, dass der Träger vor dem Aufbringen der Referenzkeime im Wesentlichen steril ist, d.h. dass er von Mikroorganismen frei ist. Frei bedeutet im Sinne der Erfindung, dass auf dem Träger ein Mikroorganismus mit einer Wahrscheinlichkeit von 10⁻¹, bevorzugt 10⁻², besonders bevorzugt 10⁻³, ganz besonders bevorzugt 10⁻⁴ oder kleiner vorhanden ist. Es kann auch bevorzugt sein, dass auf dem Träger ein Mikroorganismus mit einer Wahrscheinlichkeit von 10⁻⁵, 10⁻⁶ oder kleiner vorhanden ist.

Es kann auch bevorzugt sein, dass der Träger die nach innen zeigende Seite der Membran ist. Nach innen zeigend meint bevorzugt die Seite der Membran, auf welcher sich die Referenzkeime vor der Prüfung befinden.

Unter Referenzkeime sind bevorzugt Mikroorganismen zu verstehen, die kontrolliert und ohne Gesundheitsrisiko für das beteiligte Fachpersonal auf den Träger aufgebracht werden können. Kontrolliert bedeutet, dass beim Aufbringen eine gewünschte Keimdichte erreicht werden kann. Es kann bevorzugt sein, eine Keimdichte von beispielsweise bevorzugt mindestens 10⁷ aufzubringen. Es kann auch bevorzugt sein eine Keimdichte von bis zu 10⁷ aufzubringen. Gleichzeitig sollte ihre Anzahl bzw. Keimdichte bevorzugt unter dem zu prüfenden Desinfektionsverfahren in vorhersagbarer und fehlerfrei überprüfbarer Weise reduziert werden. Darunter ist bevorzugt zu verstehen, dass eine Keimreduktion um einen Faktor von mindestens 10³, bevorzugt 10⁴, besonders bevorzugt 10⁵ und ganz besonders bevorzugt 10⁶ stattfindet. Es können thermische oder nicht-thermische Desinfektionsverfahren mittels Referenzkeimen überprüft werden.

Bevorzugt ist, dass der Referenzkeim im Falle einer möglichen Exposition nur geringes pathogenes Risiko aufweist. Exposition bedeutet vor allem, dass eine Person mit dem Referenzkeim in direkten Kontakt kommt und ihn dabei aufnimmt. Eine Aufnahme kann insbesondere oral, inhalativ, dermal, intravenös, intramuskulär oder intraperitoneal erfolgen. Es ist bevorzugt, dass die Referenzkeime auf ähnliche Weise auf den Desinfektionsvorgang reagieren wie die typischerweise natürlich auftretenden und zu desinfizierenden Mikroorganismen bzw. mit ihnen identisch sind. Bevorzugt sind mit Referenzkeimen Bakterien, Pilze und/oder Viren gemeint. Bevorzugte Beispiele für Bakterien, Viren und/oder Pilze sind: Aspergillus fumigatus, Bacillus subtilis, Bacteroides fragilis, Candida albicans, Candida krusei, Candida tropicalis, Corynebacterium renale, Escherichia coli, Enterobacter casseluiflavus, Enterobacter hormeachei, Enterococcus faecium, Haemophilus influenzae, Klebs pneumoniae, Micrococcus luteus, Enterococcus faecalis, Neisseria gonorrhoeae, Proteus mirabilis, Proteus vulgaris, Pseudomonas aeruginosa, Staphylococcus epidermidis, Staphylococcus aureus, Streptococcus pneumoniae, Streptococcus agalactiae, Mycoplasma hominis, Pseudomonas aeruginosa, Salmonella enterica, Staphylococcus saprophyticus, Streptococcus pyogenes und/oder Ureaplasma parvum.

Unter Membran ist insbesondere eine dünne Schicht eines Materials gemeint, welche den Stofftransport durch diese Schicht beeinflusst. Die Semipermeabilität kann vor allem durch die Struktur der Membran, insbesondere durch die Geometrie von Aussparungen bzw. Poren in der Struktur erreicht werden. Diese können beispielsweise so klein sein, dass sie nicht von Referenzkeimen passiert werden können.

Unter semipermeable Membran ist bevorzugt eine Membran gemeint, welche durchlässig für Wasser und in Wasser gelöste Desinfektionswirkstoffe ist, jedoch undurchlässig für die Referenzkeime.

Bevorzugt können Membran Polymermembranen oder keramische Membranen sein. Beispiele für bevorzugte Gruppen von Materialien zur Herstellung von Membranen sind Zeolithe und/oder Polyamide.

Die semipermeable Membran ist bevorzugt mechanisch, chemisch und thermisch stabil, gleichzeitig flexibel.

Es ist bevorzugt, dass die semipermeable Membran des Bioindikators Poren aufweist, bevorzugt muss die Größe der Poren kleiner sein als der Durchmesser der abzutrennenden Bestandteile, welche bevorzugt die Referenzkeime sind. Mit Größe der Poren ist bevorzugt der Durchmesser der Poren gemeint. Die Poren können beispielsweise eine Größe von 0,5 µm aufweisen.

Es werden bevorzugt je nach Anwendung unterschiedliche Membrangeometrien verwendet. Es kann bevorzugt sein, eine Flachmembran zu verwenden, d.h. bevorzugt, dass poröse Folien aus Polymer oder keramische Scheiben, welche gerakelt oder gegossen werden, als Membrangeometrie verwendet werden. Beim Rakeln werden Polymerlösungen bevorzugt mittels einer Metall-Rakel ausgestrichen und durch eine Phaseninversion zu einer Flachmembran gefällt. Als Phaseninversion wird eine Umkehr der Phase bezeichnet. Die Phase bezeichnet in erster Linie die in einem räumlichen Bereich, welcher eine homogene chemische Zusammensetzung aufweist, homogen vorliegenden bestimmenden physikalischen Parameter, wie bspw. den Aggregatzustand. Mit Fällung oder Präzipitation wird bevorzugt in der Chemie das Ausscheiden des in einer Lösung gelösten Stoffes bezeichnet. Solche Membranen sind besonders kostengünstig, dabei effektiv. Es kann auch bevorzugt sein, kapillarartige Hohlfasermembranen zu verwenden. Unter einer Hohlfaser versteht man in der Hauptsache eine Faser, welche zylinderförmig ist und einen oder mehrere durchgängige Hohlräume in ihrem Querschnitt aufweist. Im Allgemeinen versteht man unter kapillarartig Röhrchen oder Hohlräume mit sehr kleinen Innendurchmessern, wodurch physikalische Effekte, insbesondere der Kapillareffekt, auftreten. Physikalische Begriffe wie "Kapillareffekt" sind insbesondere so zu verstehen, wie in der Fachliteratur, beispielsweise in "H. Schubert: Kapillarität in porösen Feststoffsystemen, Springer Verlag, Berlin" beschrieben wird. Solche Membranen sind bezüglich ihrer Filterfunktion besonders leistungsstark. Es kann auch bevorzugt sein, sogenannte Wickelmodule als Membran zu verwenden, d.h. bevorzugt, dass zwei Flachmembranlagen, die durch ein Gewebe voneinander getrennt spiralförmig aufgewickelt werden, verwendet werden. Auf diese Weise können besonders zuverlässig abzutrennende Bestandteile unterschiedlichster Größen gefiltert werden. Es kann auch bevorzugt sein, Multikanalelemente zu verwenden, d.h. bevorzugt, extrudierte, keramische Zylinder oder Platten, welche durch innen beschichtete Kanäle durchströmt werden. Extrusion beschreibt vor allem Vorgänge, bei denen feste bis dickflüssige, härtbare Materialien unter Druck aus einer formgebenden Öffnung herausgedrückt werden. Solche Membranen sind besonders zuverlässig. Bevorzugt können auch Composite-Membran zum Einsatz kommen. Bei diesen Membranen wird bevorzugt auf eine poröse Trägerschicht eine aktive Membranschicht aufgetragen. Unter aktive Membranschicht kann bevorzugt eine Membranschicht gemeint sein, welche außer durch ihre Geometrie auch durch zusätzliche Effekte, beispielsweise durch chemische Prozesse, eine Filterfunktion erfüllt. Es kann aber auch vorteilhafterweise gemeint sein, dass Energie hinzugeführt werden muss, um die Filterfunktion der Membran zu realisieren. Solche Membranen können eine Verbesserung der Filtercharakteristik bewirken und weisen oftmals synergistische Effekte auf, da ihre Filtereigenschaften besser sind, als allein durch die Summe der durch die Geometrie verursachten Filterfunktion und der durch die zusätzlichen Effekte verursachten Filterfunktion zu erwarten ist.

Ein Träger, der durch eine semipermeable Membran zumindest teilweise umschlossen ist, bezeichnet bevorzugt einen Träger, der von Membran wie von einer Hülle allseitig umgeben ist, so dass die von ihm getragenen Referenzkeime nicht auf die Außenseite der Membran gelangen können. Es kann auch bevorzugt sein, dass der Träger die Innenseite der Membran selber ist, so dass der von der Membran umschlossene Träger eine von der Membran bereitgestellte Fläche auf der Innenseite der Membran ist, welche von den Teilen der Membran, welche nicht zu dieser Fläche gehören, wie von einer Hülle allseitig umgeben ist. Erfindungsgemäß kann es sich hierbei auch um ein Baumwollsäckchen handeln. Es ist dabei bevorzugt, dass sich die Membran der Form des Trägermaterials dabei weitgehend anpasst. So kann ein besonders kompakter und platzsparender Bioindikator hergestellt werden.

Das Verschließen der Membran nach außen hin kann bevorzugt durch ein Schweiß- oder Klebeverfahren realisiert werden. Schweißen meint bevorzugt ein Verfahren, bei dem Wärmezufuhr bis zum Schmelzen des Werkstoffs oder Wärmezufuhr und bevorzugt zusätzlich eine Druckeinwirkung auf das Werkstück erfolgen.

Durch diese bevorzugte Ausführungsform des Bioindikators wird eine besonders einfache, robuste und kostengünstig herzustellende Vorrichtung bereitgestellt. Es kann so eine besonders einfache Herstellung des Bioindikators erfolgen, bei der zusätzlich Material eingespart wird.

Die Erfindung löst auf überraschende Weise die sich aus den Nachteilen des Standes der Technik ergebende Aufgabe. Die mikrobiologische Prüfung von Waschmaschinen mittels Bioindikator kann ohne Fachpersonal umgesetzt werden. Dabei kann es nicht dazu kommen, dass aufgrund eines fehlerhaften Desinfektionsvorgangs das gesamte Waschwasser mit pathogenen Mikroorganismen verseucht wird. Ebenso wenig kann es zu fehlerhaften Prüfergebnissen kommen, weil die Referenzkeime während des Waschvorgangs abgewaschen aber nicht unschädlich gemacht werden. Auch können die bevorzugten Bioindikatoren nicht durch bei falscher Handhabung nachträglich aufgebrachten Keimen zu fehlerhaften Prüfergebnissen führen. Es war überraschend, dass all die Nachteile bekannter Bioindikatoren durch die bevorzugte Ausführungsform überwunden werden konnten. Durch die erhöhte Sicherheit und Zuverlässigkeit bei der mikrobiologischen Prüfung von Waschmaschinen sowie der einfachen Handhabung des Bioindikators kann eine nachhaltige und kostensparende Prüfung erfolgen, welche zu keinen Unterbrechungen der täglichen Routinewaschvorgänge führt.

In einer bevorzugten Ausführungsform sind die Referenzkeime des Bioindikators ausgewählt aus dem Stamm der Firmicutes. Es ist besonders bevorzugt, einen Bioindikator bereitzustellen, dessen Referenzkeime ausgewählt sind aus der Familie der Enterokokken und/oder Staphylokokken. Enterokokken sind bevorzugt grampositive, Katalase-negative und aerotolerante, anaerobe Bakterien. Die kugelförmigen (kokkoiden) Bakterien sind in Paaren oder kurzen Ketten angeordnet. Enterokokken sind bevorzugt folgende Arten: Enterococcus avium, Enterococcus casseliflavus, Enterococcus durans, Enterococcus faecalis, Enterococcus faecium, Enterococcus gallinarum, Enterococcus hirae, Enterococcus raffinosus. Staphylokokken sind bevorzugt rundliche, weintraubenähnlich angeordnete, nicht sporenbildende grampositive Bakterien ohne aktive Bewegung aus der Gruppe der Kokken. Es können sowohl koagulasepositive Staphylokokken als auch koagulasenegative Staphylokokken bevorzugt sein. Koagulasepositive Staphylokokken sind bevorzugt Staphylococcus aureus, Staphylococcus agnetis, Staphylococcus delphini, Staphylococcus hyicus, Staphylococcus intermedius, Staphylococcus lutrae, Staphylococcus pseudintermedius und/oder Staphylococcus schleiferi subsp. Coagulans. Koagulasenegative Staphylokokken sind bevorzugt Staphylococcus epidermidis, Staphylococcus haemolyticus, Staphylococcus lugdunensis und/oder Staphylococcus saprophyticus subsp. Saprophyticus.

Es ist besonders bevorzugt, Referenzkeime ausgewählt aus der Art Enterococcus faecium und/oder Staphylococcus aureus zu verwenden. Referenzkeimen aus dieser Art sind besonders geeignet, sowohl für thermoresistente als auch nicht-thermoresistente Keime die Reduktion beim Desinfektionsvorgang gut abzubilden. Durch diese Referenzkeime kann eine mikrobiologische Prüfung von Waschmaschinen vorgenommen werden, durch welche der Desinfektionsvorgang auch für andere im klinischen Alltag vorkommende Keime auf überraschende Weise überprüft werden kann.

Referenzkeime können auch bevorzugt ausgewählt sein aus der Gruppe Mycobacterium terrae, Mycobacterium avium, Bacillus atrophaeus, Aspergillus brasiliensis, Trychophyton rubrum, Candida albicans und/oder MS 2-Phage. Durch diese Auswahl von Referenzkeimen kann eine individuell an die zu prüfende Waschmaschine und ihre Umgebung angepasste mikrobiologische Prüfung erfolgen. Beispielsweise kann es nötig sein, dass für eine Prüfung von Waschmaschinen in tropischen Ländern, in denen die Infektionsgefahr von anderen Viren und/oder Bakterien ausgeht, andere Referenzkeime sinnvoll sind als in klimatisch gemäßigten Ländern

Es kann bevorzugt sein, eine Keimdichte von mindestens 10⁸ aufzubringen. Dadurch kann eine besonders zuverlässige Prüfung durchgeführt werden. Es ist besonders bevorzugt, eine Keimdichte von mindestens 10⁷ aufzubringen. Ein solcher Bioindikator ist besonders robust gegenüber physischen Einflüssen. Es ist ganz besonders bevorzugt, eine Keimdichte von mindestens 10⁶ aufzubringen. Solch ein Bioindikator kann besonders einfach und kostengünstig hergestellt werden. Es kann auch bevorzugt sein, eine Keimdichte von bis zu 10⁸ aufzutragen. Dies hat eine Qualitätshebung der Prüfung zur Folge. Es ist besonders bevorzugt, eine Keimdichte von bis zu 10⁷ aufzubringen. Es war überraschend, dass mit so einer Keimdichte eine besonders zuverlässige Prüfung vorgenommen werden konnte. Es ist ganz besonders bevorzugt, eine Keimdichte von bis zu 10⁶ aufzubringen. Ein so hergestellter Bioindikator spart Arbeitsstufen und Zeit. Durch die bevorzugte Keimdichte kann ein individuell an den vorzunehmenden Desinfektionsvorgang angepasster Bioindikator bereitgestellt werden.

Es ist insbesondere bevorzugt, dass die Referenzkeime auf dem Träger eine Keimdichte von bis zu 10⁷ aufweisen. Mithilfe einer solchen Keimdichte lässt sich überraschend gut eine Keimreduktion aufgrund von Desinfektionsverfahren um mehrere Größenordnungen nachweisen. Typischerweise soll eine Keimreduktion um bevorzugt mindestens drei Größenordnungen (Faktor 10³) oder Log-Stufen erreicht werden. Solch eine Keimreduktion lässt sich besonders zuverlässig nachweisen. Es ist besonders bevorzugt, eine Keimreduktion um mindestens vier Größenordnungen (Faktor 10⁴) oder Log-Stufen zu erzielen. Die für diese Reduktion verfügbaren Nachweisverfahren sparen Zeit. Es ist ganz besonders bevorzugt, eine Keimreduktion um mindestens fünf Größenordnungen (Faktor 10⁵) oder Log-Stufen zu erreichen. Dies hat eine Beseitigung von Fehlern beim Nachweis zur Folge. In manchen Fällen ist es bevorzugt, das eine Keimreduktion von mindestens sechs Größenordnungen (Faktor 10⁶) oder Log-Stufen erreicht wird. So kann ein verbesserter Nachweis erbracht werden. Bei der bevorzugten Keimdichte von bis zu 10⁷ lässt sich vorgenannte Keimreduktion ohne großen Aufwand nachweisen. Zum Nachweis der Keimreduktion können überraschenderweise gängige Verfahren, wie beispielsweise bevorzugt die Gram-Färbung, lichtmikroskopische Untersuchungen, aber auch das Anwachsen einer Kultur auf einem Nährboden, verwendet werden.

Es kann bevorzugt sein, dass die Membran Poren eine Größe von > 0,1 µm zu verwenden. Diese sind besonders einfach und kostengünstig herzustellen. Es können auch Poren einer Größe von < 0,1 µm bevorzugt zum Einsatz kommen. Diese haben eine verbesserte Filterfunktion gegenüber manchen Keimen zur Folge. Es kann ebenfalls bevorzugt sein, Poren einer Größe von höchstens 2 nm zu verwenden. Diese sind für eine besonders zuverlässige Durchführung der Prüfung geeignet.

In einer bevorzugten Ausführungsform weisen die Poren der Membran eine Größe < 2 µm auf. Diese haben eine Qualitätshebung des Prüfverfahrens zur Folge. Es ist dabei besonders bevorzugt, dass die Poren eine Größe von < 1 µm haben, denn so kann die Effektivität des Prüfverfahrens gesteigert werden. Es ist insbesondere bevorzugt, dass die Poren eine Größe von < 0,6 µm aufweisen. So können Fehler beim Prüfverfahren beseitigt werden.

Es war völlig überraschend, dass durch die bevorzugte Porengröße ein Bioindikator bereitgestellt werden konnte, welcher die erfindungsgemäße Aufgabe auf einfache und zuverlässige Weise löst. Insbesondere kann durch die bevorzugte Porengröße auf einfache Weise sichergestellt werden, dass keine der bevorzugten Referenzkeime vom Inneren der Membran nach außen gelangt.

Bevorzugt können Membran Polymermembranen oder keramische Membran sein. Die Membranmaterialien können bevorzugt folgende Materialien oder Materialgruppen umfassen: Polysulfone, Polyethersulfon, Silikone, Polyamide, Polyamidimid, Polyamid Harnstoff, Polycarbonate, Zeolithe, Polyacrylnitril, Polyethylen, Polypropylen, Polytetrafluorethylen, Polyvinylidenfluorid, Polyvinylchlorid und/oder Polypiperazinamid. Diese Materialien eignen sich für eine besonders kostengünstige Herstellung. Ebenso erhöhen diese Materialien überraschenderweise den Filtereffekt. Dadurch kann bezüglich der Filterfunktion ein synergistischer Effekt erzielt werden, da die Materialien selber, unabhängig von der Membran, die Filterfunktion verbessern.

Die Membran können ebenfalls bevorzugt Cellulose, Celluloseester und/oder regenerierter Cellulose umfassen. So können besonders einfach auch recycelte Materialien zum Einsatz kommen und nachhaltig Ressourcen und Kosten gespart werden.

Es kann auch bevorzugt sein, dass die Membran Keramik und/oder Edelstahl umfasst. Mit diesen Materialien kann eine Membran mit einer besonders leistungsstarken Filterfunktion bereitgestellt werden.

Weiterhin kann bevorzugt sein, dass die Membran Silber und/oder Silizium umfasst. Durch diese Materialien kann die Membran verbessert werden.

Durch die bevorzugten Materialien zur Membranherstellung kann eine Membran bereitgestellt werden, die sowohl zur Lösung der erfindungsgemäßen Aufgabe beiträgt, als auch sehr einfach herzustellen ist. Insbesondere in Kombination mit der bevorzugten Porengröße ergeben sich synergistische Effekte, die auf überraschende Weise dazu beitragen, dass kein Referenzkeim vom Inneren der Membran nach außen gelangen kann. Es war völlig überraschend, dass durch die Kombination von bevorzugtem Material und bevorzugter Porengröße diese Filterfunktion der Membran sogar funktionieren kann, wenn die Referenzkeime kleiner sind als die Porengröße.

Es ist bevorzugt, dass der Träger eine flächige Form aufweist. Unter flächig ist bevorzugt zu verstehen, dass das Element in zwei Dimensionen eine größere Ausdehnung als in einer dritten Dimension aufweist. Die Träger können bevorzugt im Wesentliche rund oder eckig sein. Es kann bevorzugt sein, dass die Träger dreieckig, viereckig, quadratisch oder rechteckig sind, aber auch Fünfecke oder Sechsecke können bevorzugt sein. Es ist bevorzugt, dass die Form im Wesentlichen ein Polygon ist. Durch die Anpassung der Form an die Anwendung kann ein besonders vorteilhaftes Prüfverfahren durchgeführt werden.

Durch die bevorzugte, flächige Form kann auf überraschende Weise ein Bioindikator zur Verfügung gestellt werden, welcher den Waschvorgang bei der mikrobiologischen Prüfung von Waschmaschinen nicht stört. Auch bei vollen Waschmaschinen wird durch die Beschickung der Waschmaschine mit dem Bioindikator keine Beeinträchtigung des Waschvorgangs hervorgerufen. Insbesondere in Kombination mit der bevorzugten, flexiblen Ausführungsform ergibt sich ein synergistischer Effekt und die Anpassungsfähigkeit an den Beladungszustand der Waschmaschine kann in stärkerer Weise verbessert werden, als es durch die einzelne Betrachtung der Form und der Flexibilität des Indikators zu erwarten war. Somit ergibt sich bezüglich der Beladung der Waschmaschine mit Wäsche oder Waschgut sowie bezüglich der Güte des Waschvorgangs bei der Beschickung der Waschmaschine mit dem Bioindikator kein Unterschied zu einem normalen Waschvorgang ohne Bioindikator.

Eine Waschmaschine im Sinne der Erfindung umfasst bevorzugt alle Maschinen, die einen Desinfektionsvorgang durchführen bzw. nachweisen können. Hierbei kann es sich bspw. auch um Spül- oder andere -maschinen und -vorrichtungen handeln.

Es ist bevorzugt, dass das Trägermaterial textiles Material oder textile Materialien umfasst. Es können bevorzugt Naturfasern wie Steinwolle, Baumwollfasern, Flachsfasern, Hanffasern oder tierische Fasern wie z. B. Wolle, Seide oder Fellhaare zum Einsatz kommen. Diese weisen sich durch eine hohe Zuverlässigkeit sowie geringe Herstellungskosten aus. Außerdem können diese Trägermaterialien überraschenderweise die Filterfunktion der Membran verbessern. Es kann aber auch bevorzugt sein, dass Chemiefasern wie Viskose, Lyocell, Gummi oder aus synthetischen Polymeren wie z. B. Polyacrylnitril, Polypropylen, Polyester, Polyamid oder Polyurethan zum Einsatz kommen. Diese lassen sich besonders leicht verarbeiten und sparen Arbeitsstufen bei der Herstellung ein. Es können auch anorganische Fasern wie z. B. Keramik-, Glas- oder Metallfasern verwendet werden. Diese zeichnen sich durch eine besondere Effektivität als Trägersubstanz aus. Es kann auch bevorzugt sein, dass der Träger mit der Innenseite der Membran identisch ist oder zumindest aus dem gleichen Material besteht. So können Materialien eingespart und Kosten reduziert werden. Das Trägermaterial umfasst in diesem Fall bevorzugt folgende Materialien oder Materialgruppen: Polysulfone, Polyethersulfon Cellulose, Celluloseester, Regenerierte Cellulose, Silikone, Polyamide, Polyamidimid, Polyamid Harnstoff, Polycarbonate, Keramik, Edelstahl, Silber, Silizium, Zeolithe, Polyacrylnitril, Polyethylen, Polypropylen, Polytetrafluorethylen, Polyvinylidenfluorid, Polyvinylchlorid und/oder Polypiperazinamid.

Durch diese bevorzugte Ausführungsform des Trägers ergibt sich eine hohe Flexibilität bezüglich der Herstellung des Bioindikators. Besonders die bevorzugte Variante, bei der der Träger aus Naturfasern, wie beispielsweise Baumwolle besteht, führt zu einem besonders einfachen, robusten und kostengünstig herstellbaren Bioindikator.

In einem weiteren Aspekt betrifft die Erfindung ein Kit (1), aufweisend 4 bis 16, bevorzugt 7 bis 12, besonders bevorzugt 8 bis 11, insbesondere 10 Bioindikatoren (2) nach einem oder mehreren der vorigen Ansprüche, sowie bevorzugt umfassend einen Anwendungsbogen und/oder einen Begleitbogen und/oder mindestens einen Versandbeutel.

In einem weiteren Aspekt betrifft die Erfindung also auch einen Kit, welches 4 bis 16 Bioindikatoren zur mikrobiologischen Überprüfung von Waschmaschinen aufweist. Diese Anzahl von Bioindikatoren verbessert die mikrobiologische Überprüfung. Es ist bevorzugt, dass das Kit 7 bis 12 Bioindikatoren aufweist. Bei einer solchen Anzahl kann die Qualität der Überprüfung gehoben werden. Es ist dabei besonders bevorzugt, dass das Kit 8 bis 11 Bioindikatoren aufweist. Eine solche Anzahl von Bioindikatoren erhöht die Effektivität. Insbesondere bevorzugt ist ein Kit, welches 10 Bioindikatoren zur mikrobiologischen Überprüfung von Waschmaschinen aufweist. Mit einem solchen Kit kann eine besonders zuverlässige Überprüfung durchgeführt werden. Das Kit umfasst dabei bevorzugt einen Anwendungsbogen, einen Begleitbogen und/oder mindestens einen Versandbeutel. Anwendungsbogen beschreibt bevorzugt eine Handlungsanweisung zur Durchführung des Prüfverfahrens. Begleitbogen beschreibt bevorzugt ein von der prüfenden Person auszufüllendes Formular zur Qualitätssicherung, in welches bevorzugt beispielsweise das Datum, Daten zur Zuordnung der geprüften Waschmaschine wie beispielsweise Standort, der Name und die Unterschrift des Prüfers und/oder das durchgeführte Waschprogramm eingetragen werden. Versandbeutel bezeichnet bevorzugt einen Beutel, der die sichere Versendung des Kits auf postalischem Wege ermöglicht. Dabei ist unter sicher zu verstehen, dass das Kit zerstörungsfrei und ohne wesentlichen Einfluss auf die Keimdichte verbleibt. In dieser bevorzugten Ausführungsform umfassend 4 bis 16, bevorzugt 7 bis 12, besonders bevorzugt 8 bis 11, insbesondere 10 Bioindikatoren, kann auf besonders zuverlässige Weise geprüft werden, ob ein Desinfektionsverfahren den Sicherheitsstandards genügt. Sollte ausnahmsweise einer oder gar mehrere der Bioindikatoren nicht ordnungsgemäß funktionieren, zum Beispiel, weil durch einen Fehler bei der Präparierung nicht die richtige Keimdichte von Referenzkeimen aufgebracht wurde oder weil die Poren der Membran verstopft wurden, können über die übrigen Bioindikatoren trotzdem zuverlässige Aussagen getroffen werden. Des Weiteren können über die Verwendung verschiedener Referenzkeime, beispielsweise von 5 Bioindikatoren mit den Referenzkeimen der Gruppe Enterococcus faecium und fünf Bioindikatoren mit den Referenzkeimen der Gruppe Staphylococcus aureus bessere Aussagen über den Erfolg des Desinfektionsverfahrens für verschiedenste Keime gemacht werden. Durch die bevorzugte Ausführungsform umfassend ebenfalls bevorzugt einen Anwendungsbogen, einen Begleitbogen und/oder mindestens einen Versandbeutel wird die Anwendung auch für nicht fachmännische Benutzer erleichtert. Insbesondere können die gewöhnlicherweise mit dem Wasch- oder Desinfektionsvorgang betrauten Fachkräfte ebenso die Benutzung der Bioindikatoren zur Prüfung der Desinfektionsleistungen der Waschmaschinen, mit denen die Desinfektion durchgeführt werden soll, z.B. Waschmaschinen durchführen. Durch den Bioindikator in der bevorzugten Ausführungsform sowie den zusätzlichen bevorzugten Anwendungs- und/oder Begleitbogen ist die Durchführung der mikrobiologischen Prüfung leicht und fehlerfrei handhabbar. Die eigentliche Überprüfung der Desinfektionsleistung kann von einem dritten Dienstleister oder vom Hersteller des Bioindikators vorgenommen werden. Dazu werden die Bioindikatoren einfach mittels des bevorzugt umfassten mindestens einen Versandbeutels eingesendet. Durch diese einfache Handhabung kann ein sehr zuverlässiges Prüfverfahren gewährleistet werden und eine versehentliche oder mutwillige falsche Durchführung des Prüfverfahrens wird vermieden. Durch die einfache Handhabung des Prüfverfahrens wird eine überraschend hohe Compliance, d. h. bevorzugt eine hohe Wahrscheinlichkeit der Einhaltung der bevorzugten Prüfabstände sowie Prüfabläufe, gewährleistet. Darüber hinaus können die Bioindikatoren eines solchen Kits, welche geeignete, hier beschriebene Merkmale aufweisen, den Vorschriften bzw. Richtlinien zahlreicher Länder, wie bspw. Deutschland oder Österreich, bezüglich der hier relevanten Prüfverfahren genügen. So kann eine Verbesserung des Prüfverfahrens durch die Benutzung eines solchen Kits erzielt werden, da der Benutzer gleichzeitig die eigene Qualitätskontrolle sowie die ggf. staatlich vorgeschriebene Überprüfung durchführen kann. Kosten und Arbeitsstufen können so gespart werden. Da die Vorschriften und Richtlinien sich an langjährigen Erfahrungen der Behörden orientieren und somit die Praxis der hier relevanten Überprüfungen abbilden, kann die Sicherheit zusätzlich erhöht werden. Des Weiteren wird durch die hier vorgestellten Bioindikatoren zur Erfüllung der Vorschriften und/oder Richtlinien ein neuer Weg beschritten.

Es kann bevorzugt sein, dass das Kit des Weiteren mindestens ein Baumwollsäckchen umfasst, in welches die Bioindikatoren während des Waschvorgangs eingebracht werden können. Dies kann zu einer zusätzlichen Verbesserung der Filterfunktion der Membran beitragen und somit die Sicherheit und Zuverlässigkeit des Verfahrens erhöhen. Darüber hinaus können so die Waschmaschine und das Waschgut geschont und somit langfristig Kosten gespart werden.

Eine vorteilhafte Ausführungsform betrifft ein Kit, wobei das Kit 2 bis 8, bevorzugt 3 bis 6, besonders bevorzugt 4 bis 6, insbesondere 5 Bioindikatoren Referenzkeime aufweisen aus der Gruppe Enterococcus faecium und 2 bis 8, bevorzugt 3 bis 6, besonders bevorzugt 4 bis 6, insbesondere 5 Bioindikatoren Referenzkeime aufweisen aus der Gruppe Staphylococcus aureus und wobei die Anzahl der Bioindikatoren mit Referenzkeimen aus der einen Gruppe bevorzugt der Anzahl der Bioindikatoren aus der anderen Gruppe entspricht.

Es ist also bevorzugt, wenn das Kit jeweils die gleiche Anzahl an Bioindikatoren mit Referenzkeimen aus der Gruppe Enterococcus faecium und an Bioindikatoren mit Referenzkeimen aus der Gruppe Staphylococcus aureus aufweist. Durch diese bevorzugte Ausführungsformen kann auf besonders zuverlässige Weise der Erfolg von Desinfektionsverfahren überprüft werden. Es ist dabei bevorzugt, dass das Kit für jeden Referenzkeim 2 bis 8 Bioindikatoren, also insgesamt 4 bis 16 Bioindikatoren aufweist. Ein solches Kit ist besonders robust. Es ist dabei weiterhin bevorzugt, dass das Kit 3 bis 6 Bioindikatoren für jeden Referenzkeim, also insgesamt 6 bis 12 Bioindikatoren umfasst. So kann eine Erhöhung der Effektivität des Kits erreicht werden. Besonders bevorzugt ist, dass das Kit 4 bis 6 Bioindikatoren für jeden Referenzkeim, also insgesamt 8 bis 12 Bioindikatoren aufweist. Solch ein Kit trägt zur Fehlerbeseitigung bei der Überprüfung bei. Insbesondere ist bevorzugt, dass das Kit 5 Bioindikatoren für jeden Referenzkeim, also insgesamt 10 Bioindikatoren, aufweist. Somit ist es auch möglich, einen Kit mit bspw. 10 Bioindikatoren einzusetzen mit jeweils der gleichen Anzahl an Bioindikatoren mit Referenzkeimen aus der Gruppe Enterococcus faecium und Referenzkeimen aus der Gruppe Staphylococcus aureus. Überraschenderweise hat sich herausgestellt, dass ein solches Kit zu einer besonders zuverlässigen Überprüfung von Waschmaschinen führt. Darüber hinaus kann es besonders kostengünstig hergestellt werden. Die Produktion eines solchen Kits kann besonders gut rationalisiert werden. Insbesondere sprechen Referenzkeimen aus der Gruppe Enterococcus faecium und Staphylococcus aureus auf unterschiedliche Aspekte eines Desinfektionsverfahrens an. Beispielsweise besitzt Enterococcus faecium eine recht hohe Thermoresistenz und kann daher insbesondere als Repräsentant thermoresistenter Keime im Prüfverfahren angesehen werden. Beide Gruppen von Keimen decken in ihrem Ansprechverhalten bei einem Desinfektionsverfahren das Verhalten einer Vielzahl von Keimen ab. Es war völlig überraschend, dass sich durch die bevorzugte Verwendung von je fünf Bioindikatoren mit Referenzkeimen aus der Gruppe Enterococcus faecium bzw. Staphylococcus aureus eine äußert effektive, zuverlässige und kostengünstige Überprüfung von Desinfektionsverfahren für Waschmaschinen realisieren ließ.

Es ist vorteilhaft, wenn das Kit an der Außenfläche klar erkenntlich eine eindeutig zuordenbare Nummer trägt, die beispielsweise bevorzugt durch einen Stempel aufgebracht wurde. Durch die Nummer kann das Kit schnell und eindeutig identifiziert werden. Mit dieser Nummer können bevorzugt beispielsweise Daten wie die zu überprüfende bzw. überprüfte Desinfektionsstelle, die verwendeten Referenzkeime sowie ihre Dichte, das Herstellungsdatum des Bioindikators, etc., verknüpft sein.

Durch diese bevorzugte Ausführungsform kann auf überraschende Weise eine fehlerhafte Zuordnung des Kits vermieden werden. Die Nummer kann bevorzugt als redundantes Identifikationsmerkmal neben dem ausgefüllten Begleitbogen angesehen werden. So kann vermieden werden, dass ein aus einer mikrobiologischen Prüfung einer Waschmaschine mithilfe des Kits resultierendes Prüfergebnis einer falschen Waschmaschine zugeordnet wird. Solch eine falsche Zuordnung könnte hohe Kosten und ein hohes gesundheitliches Risiko durch Infektionen aufgrund eines nicht funktionierenden, nicht richtig identifizierten Desinfektionsvorgangs zur Folge haben.

Es ist bevorzugt, dass die einzelnen Bioindikatoren des Kits zusammengefasst in einem flächigen Element vorliegen. Ein solches Kit trägt zur Materialersparnis bei. Die Form des Elements ist im Wesentlichen bevorzugt rechteckig. Ein solches Element ist überraschenderweise besonders gut geeignet, einen reibungslosen Waschgang während der Prüfung zu gewährleisten. Das Element ist bevorzugt sichtbar unterteilt in die einzelnen Bioindikatoren. Das trägt zur Sicherheit und Zuverlässigkeit des Prüfverfahrens bei, insbesondere können Fehler bei der Herstellung der Bioindikatoren leichter entdeckt werden. Es ist bevorzugt, dass das Element mechanisch stabil, dennoch flexibel ist. Ein solches Element schont die zu überprüfende Waschmaschine sowie das Waschgut und trägt so zur Reduktion von Kosten bei. Bevorzugt ist an einer Randfläche des Elements die Zuordnungsnummer sichtbar. Das erhöht zusätzlich die Sicherheit und Zuverlässigkeit, Fehler können besser behoben werden. Sowohl in der bevorzugten Ausführungsform, bei der der Träger die Innenseite der Membran selber ist, als auch bei der bevorzugten Ausführungsform, wo der Träger textiles Material oder textile Materialien aufweist, kann es bevorzugt sein, dass die einzelnen Bioindikatoren durch Schweißnähte der Membran voneinander getrennt sind. So kann das Überprüfungsverfahren verbessert werden, es kann nicht zu einer gegenseitigen Kontamination der Bioindikatoren kommen. Es kann bevorzugt sein, an einigen Stellen doppelte Schweißnähte zwischen den Bioindikatoren einzufügen, zwischen denen kleine, nicht verschweißte, flächige Elemente entstehen. Diese doppelten Schweißnähte sowie die kleinen, flächigen Elemente können zur klaren Kennzeichnung der verschiedenen Bioindikatoren, beispielsweise zur Unterscheidung der von Ihnen getragenen Referenzkeime, verwendet werden. Dies erhöht die Zuverlässigkeit insbesondere bei der Kontrolle der Keimreduktion. Es kann auch bevorzugt sein, dass die kleinen, flächigen Elemente nicht Träger von Referenzkeimen sind und somit zur Kontrolle der Funktionsfähigkeit des Bioindikators herangezogen werden können. So können Fehler behoben werden. Es kann zur Kontrolle der Funktionsfähigkeit auch bevorzugt sein, dass sie Referenzkeime tragen, aber nicht von einer semipermeablen Membran, sondern einer undurchlässigen Membran umgeben sind. So kann die Zuverlässigkeit des Verfahrensüberprüft und erhöht werden. Es können auch beide Funktionalitäten der kleinen, flächigen Elemente zugleich bevorzugt Anwendung finden, indem beispielsweise unterschiedliche Elemente unterschiedlich genutzt werden. Dies hat einen synergistischen Effekt bezüglich der Sicherheit und Zuverlässigkeit des Verfahrens sowie der Beseitigung von Fehlern zur Folge, da sich diese Aspekte in einem Maße verbessern, welches über das Maß hinausgeht, welches die einzelnen Anwendungen der kleinen flächigen Elemente für sich genommen erwarten lassen.

In einem weiteren Aspekt betrifft die Erfindung auch ein Verfahren zur mikrobiologischen Prüfung von Waschmaschinen, umfassend bevorzugt folgende Schritte: die Entnahme mindestens eines Bioindikators aus einem Versandbeutel, die Beschickung einer zu prüfenden Waschmaschine mit dem mindestens einen Bioindikator, bevorzugt gemeinsam mit einem zu desinfizierenden und/oder zu waschenden Gut, das Ausfüllen des Begleitbogens, die Entnahme und vorzugsweise die Trocknung des mindestens einen Bioindikators aus der Waschmaschine nach dem Desinfektionsvorgang, und schließlich das Einlegen des mindestens einen Bioindikators und des Begleitbogens in einen Versandbeutel zum Zwecke seiner Rücksendung sowie der Rücksendung des Bioindikators und des Begleitbogens im Versandbeutel. Das bevorzugte Kit wird in einem Versandbeutel, welcher zum sicheren Versenden geeignet ist, an die Prüfstelle bevorzugt postalisch zugestellt. Der Versandbeutel kann dabei bevorzugt auch zur sicheren Lagerung des Kits verwendet werden. Vor der Prüfung kann das Kit bevorzugt dem Versandbeutel entnommen werden. Es kann bevorzugt sein, dass ein Begleitbogen sowie ein bevorzugt vorhandener, zweiter Versandbeutel zur Rücksendung ebenfalls dem Versandbeutel beiliegt und entnommen werden kann. Zur Prüfung wird die zu prüfende Maschine, bspw. die Spül- oder Waschmaschine, mit dem Kit beschickt. Dabei kann es bevorzugt sein, dass Kit einzeln oder gemeinsam mit Wäsche der Waschmaschine zugefügt wird. Im zweiten Fall kann es bevorzugt sein, das Kit lose oder mit der Wäsche wirkverbunden zuzufügen. Unter wirkverbunden kann bevorzugt beispielsweise die Verbindung des Kits mit der Wäsche mittels eines Kabelbinders, aber auch die Verbindung durch Einwickeln in die Wäsche oder durch Einstecken in einer in der Wäsche vorhandenen Tasche gemeint sein. Nach der Einstellung des zu prüfenden Waschprogramms, der Zugabe des bevorzugt zu verwendenden Waschmittels und dem Starten des Waschvorgangs kann bevorzugt das Ausfüllen des Begleitbogens durch die prüfende Person vorgenommen werden. Dabei sind bevorzugt für die Identifizierung der geprüften Waschmaschine relevante Daten, das Datum, der Name und die Unterschrift der prüfenden Person, das gewählte Waschprogramm und andere eventuell relevante Informationen und Anmerkungen einzutragen. Das Ausfüllen des Begleitbogens kann natürlich auch zu einem späteren Zeitpunkt vor der Rücksendung vorgenommen werden. Nach Beendigung des Waschvorgangs wird der Bioindikator der Waschmaschine entnommen, bevorzugt getrocknet, und dann gemeinsam mit dem Begleitbogen in einen Versandbeutel eingelegt. Dabei kann es sich bevorzugt um einen neuen, ebenfalls mitgelieferten Versandbeutel, aber auch um den gleichen Versandbeutel wie der zur vorherigen Zustellung verwendete handeln. Bevorzugt trägt der Versandbeutel bereits die Adresse des Dienstleisters bzw. der Prüfstelle zur Überprüfung der Keimreduktion. Dann kann der Versandbeutel samt Inhalt versendet werden.

Es kann bevorzugt sein, dass bei der Beschickung der zu prüfenden Waschmaschine mit mindestens einen Bioindikator dieser vorher in ein Baumwollsäckchen eingebracht wird, um die Sicherheit und Zuverlässigkeit des Verfahrens zu erhöhen und die Kosten zu senken.

Der mindestens eine Bioindikator dieses Verfahrens kann vorteilhafterweise auch als Element umfassend mehrere Bioindikatoren vorliegen und/oder die in einem Kit enthaltenen Bioindikatoren umfassen.

Durch diese bevorzugte Ausführungsform kann ein überraschend sicheres, zuverlässiges, effizientes, kostengünstiges und einfach durchzuführendes Verfahren zur mikrobiologischen Überprüfung von z. B. Waschmaschinen bereitgestellt werden. Es war völlig überraschend, dass durch die vorangestellten Verfahrensschritte eine äußerst hohe Compliance bei der Durchführung der Prüfung der Desinfektionsleistung von Waschmaschinen gewährleistet werden konnte. Auch nicht sachkundiges Personal, welches zum Teil unter hohem Zeitdruck arbeitet, und für welches die Überprüfung der Desinfektionsleistung eine lästige Zusatzarbeit bedeutet, wurde durch vorangestelltes Verfahren in die Position gebracht, fehlerfrei und ohne große Zeitverluste ein Verfahren zur mikrobiologischen Überprüfung von Waschmaschinen durchzuführen. Darüber hinaus kann das Verfahren unter Verwendung geeigneter Bioindikatoren den Vorschriften bzw. Richtlinien zahlreicher Länder, wie bspw. Deutschland oder Österreich, bezüglich der relevanten Prüfverfahren genügen. So kann eine Verbesserung des Prüfverfahrens erzielt werden, da der Benutzer gleichzeitig die eigene Qualitätskontrolle sowie die ggf. staatlich vorgeschriebene Überprüfung durchführen kann. Kosten und Arbeitsstufen können so gespart werden. Da die Vorschriften und Richtlinien sich an langjährigen Erfahrungen der Behörden orientieren und somit die Praxis von den hier relevanten Überprüfungen abbilden, kann die Sicherheit zusätzlich erhöht werden. Des Weiteren wird durch die hier vorgestellten Bioindikatoren zur Erfüllung der Vorschriften und/oder Richtlinien ein neuer Weg beschritten.

In einem weiteren Aspekt betrifft die Erfindung die Verwendung des Bioindikators zur mikrobiologischen Prüfung von Waschmaschinen. Der Fachmann erkennt, dass die bevorzugten Ausführungsformen und Vorteile der Vorrichtung und des Verfahrens ebenfalls für die Verwendung gelten.

### Kurzbeschreibung der Abbildung

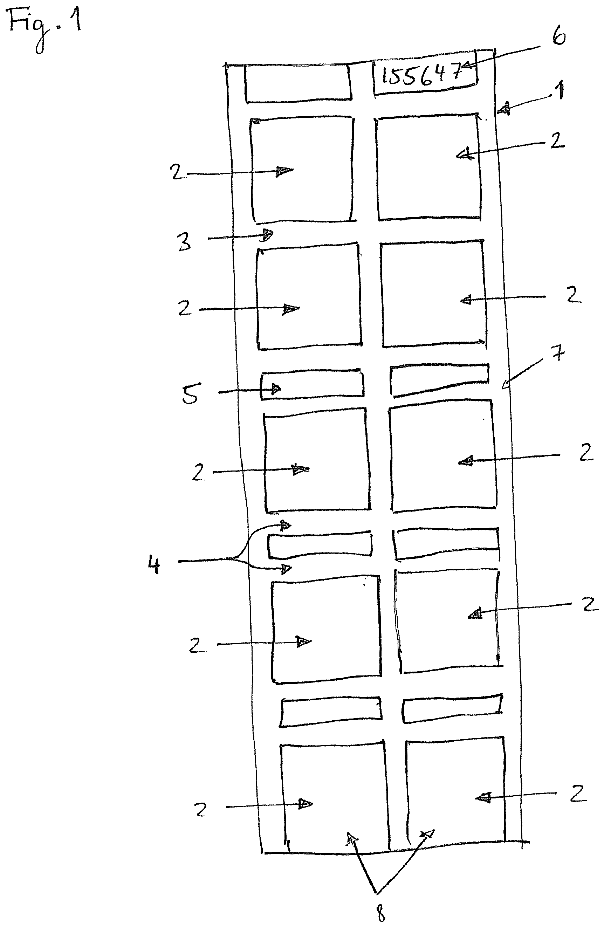

Fig. 1: Schematische Draufsicht einer bevorzugten Ausführungsform der Erfindung umfassend ein Kit, welches 10 Bioindikatoren zur mikrobiologischen Überprüfung von Waschmaschinen aufweist.

### Detaillierte Beschreibung der Abbildung

Figur 1 zeigt eine schematische Draufsicht einer bevorzugten Ausführungsform der Erfindung umfassend ein Kit (1), welches 10 Bioindikatoren (2) zur mikrobiologischen Überprüfung von Waschmaschinen aufweist. Die Schweißnähte (3) und doppelten Schweißnähte (4) liegen zwischen den Bioindikatoren bzw. den kleinen, flächigen Elementen (5) sowie an den Außenkanten (7) des Kits (1) vor. In der gezeigten, bevorzugten Ausführungsform des Kits liegen die Bioindikatoren (2) paarweise nebeneinander vor. Fünf dieser Paare (8) sind in einem Kit übereinander gruppiert. Es kann bevorzugt sein, dass jeweils ein Bioindikator (2) des Paars (8) mit einem Referenzkeim der Gruppe Enterococcus faecium und der andere mit einem Referenzkeim der Gruppe Staphylococcus aureus versehen ist. Es kann bevorzugt sein, dass immer der in der Draufsicht linke Bioindikators des Paares (8) mit einem der beiden Referenzkeime versehen ist und immer der rechte Bioindikator mit dem anderen. Eine zusätzliche Strukturierung wie in der gezeigten, bevorzugten Ausführungsform des Kits (1) ist bevorzugt. Beispielsweise ist bevorzugt, die doppelten Schweißnähte (4) und die kleinen, flächigen Elemente (5) zwischen den (in der Draufsicht) unteren drei Paaren (8) der Bioindikatoren (2) anzuordnen. So kann bei dem Bioindikator gut zwischen oben und unten, wie sie in der vorliegenden Zeichnung definiert sind, unterschieden werden. Weiterhin ist in der bevorzugten Ausführungsform am oberen Rand, bevorzugt am oberen rechten Rand, eine eindeutig zuordenbare Nummer (6) gut lesbar angebracht.

## Patentansprüche

1. Bioindikator zur mikrobiologischen Prüfung von Waschmaschinen
**dadurch gekennzeichnet, dass**
- der Bioindikator einen Träger umfasst,
- der Träger Referenzkeime aufweist, wobei
- der Träger durch eine semipermeable Membran umschlossen ist.

2. Bioindikator nach Anspruch 1, **dadurch gekennzeichnet, dass** die Referenzkeime ausgewählt sind aus der Gruppe Enterococcus faecium und/oder Staphylococcus aureus.

3. Bioindikator nach Anspruch 1 oder 2, **dadurch gekennzeichnet, dass** die Referenzkeime auf dem Träger eine Keimdichte von bis zu 10⁷ aufweisen.

4. Bioindikator nach einem oder mehreren der vorigen Ansprüche, **dadurch gekennzeichnet, dass** die Membran Poren einer Größe von < 2 µm, besonders bevorzugt < 1 µm, ganz besonders bevorzugt < 0,6 µm aufweist.

5. Bioindikator nach einem oder mehreren der vorigen Ansprüche, **dadurch gekennzeichnet, dass** die semipermeable Membran Polysulfon, Polyethersulfon, Silikonen, Polyamid, Polyamidimid, Polyamid Harnstoff, Polycarbonat, Zeolithe, Polyacrylnitril, Polyethylen, Polypropylen, Polytetrafluorethylen, Polyvinylidenfluorid, Polyvinylchlorid und/oder Polypiperazinamid umfasst.

6. Bioindikator nach einem oder mehreren der vorigen Ansprüche, **dadurch gekennzeichnet, dass** die semipermeable Membran Cellulose, Celluloseester und/oder regenerierte Cellulose umfasst.

7. Kit (1), aufweisend 4 bis 16, bevorzugt 7 bis 12, besonders bevorzugt 8 bis 11, insbesondere 10 Bioindikatoren (2) nach einem oder mehreren der vorigen Ansprüche, sowie bevorzugt umfassend einen Anwendungsbogen und/oder einen Begleitbogen und/oder mindestens einen Versandbeutel.

8. Kit (1) nach Anspruch 7, wobei 2 bis 8, bevorzugt 3 bis 6, besonders bevorzugt 4 bis 6, insbesondere 5 Bioindikatoren (2) Referenzkeime aufweisen aus der Gruppe Enterococcus faecium und 2 bis 8, bevorzugt 3 bis 6, besonders bevorzugt 4 bis 6, insbesondere 5 Bioindikatoren Referenzkeime aufweisen aus der Gruppe Staphylococcus aureus und wobei die Anzahl der Bioindikatoren mit Referenzkeimen aus der einen Gruppe der Anzahl der Bioindikatoren aus der anderen Gruppe entspricht.

9. Verfahren zur mikrobiologischen Prüfung von Waschmaschinen, umfassend die folgenden Schritte
- Entnahmen des mindestens einen Bioindikators aus einem Versandbeutel,
- Beschickung der zu prüfenden Waschmaschine mit dem mindestens einen Bioindikator, bevorzugt gemeinsam mit einem zu desinfizierenden und/oder zu waschenden Gut,
- Ausfüllen des Begleitbogens,
- Entnahme und bevorzugt Trocknung des mindestens einen Bioindikators,
- Einlegen des mindestens einen Bioindikators und des Begleitbogens in einen Versandbeutel und Rücksendung des Bioindikators und des Begleitbogens.

10. Verwendung des Bioindikators nach einem oder mehreren der Ansprüche 1 bis 6 zur mikrobiologischen Prüfung von Waschmaschinen.
